# EUROPEAN PATENT SPECIFICATION

(11) **EP 3 397 954 B1**
(45) Date of publication and mention of the grant of the patent: **03.04.2024**
(21) Application number: 16823281.7
(22) Date of filing: 22.12.2016
(51) Int. Cl.: G01N 27/327

(54) **HAND-HELD TEST METER FOR USE WITH ELECTROCHEMICAL-BASED ANALYTICAL TEST STRIP WITH ELECTRODE VOLTAGE SENSING CONNECTIONS**
TRAGBARES MESSGERÄT ZUR VERWENDUNG EINES ELEKTROCHEMISCHEN ANALYSETESTSTREIFENS MIT ELEKTRODENSPANNUNGSMESSANSCHLÜSSEN
APPAREIL DE MESURE PORTATIF À UTILISER AVEC UNE BANDELETTE D'ESSAI ANALYTIQUE DE TYPE ÉLECTROCHIMIQUE AVEC DES CONNEXIONS DE DÉTECTION DE TENSION D'ÉLECTRODE

(30) Priority: 28.12.2015 US 201562271473 P
(43) Date of publication of application: 07.11.2018
(73) Proprietor: Lifescan Scotland Limited, Inverness, Inverness-Shire IV2 3ED (GB)
(72) Inventor: ELDER, David, Inverness Inverness-shire IV2 3ED (GB); MCCOLL, David, Inverness Inverness-shire IV2 3ED (GB)
(74) Representative: Carpmaels & Ransford LLP
(86) International application number: PCT/EP2016/082357
(87) International publication number: WO 2017/114746

(56) References cited:
- WO-A1-2006/074927
- US-A1- 2006 278 538
- US-A1- 2015 330 935
- US-A1- 2015 362 455

## Description

### BACKGROUND OF THE INVENTION

### Field of the Invention

The present invention relates, in general, to medical devices and, in particular, to hand-held test meters for use with electrochemical-based analytical test strips.

### Description of Related Art

The determination (e.g., detection and/or concentration measurement) of an analyte in, or a characteristic of, a fluid sample is of particular interest in the medical field. For example, it can be desirable to determine glucose, ketone bodies, cholesterol, lipoproteins, triglycerides, acetaminophen, hematocrit and/or HbA1c concentrations in a sample of a bodily fluid such as urine, blood, plasma or interstitial fluid. Such determinations can be achieved using analytical test strips, based on, for example, visual, photometric or electrochemical techniques in conjunction with a hand-held test meter. Conventional electrochemical-based analytical test strips are described in, for example, U.S. Patent Nos. 5,708,247 and 6,284,125.

WO 2006/074927 A1 describes an electrode compensation circuit housed within a test meter. A microprocessor outputs a digital signal indicative of the voltage potential to be applied to a counter electrode contact pad. This digital signal is converted to an analog voltage signal by a D/A. The analog output of the D/A is applied to a first input of an operational amplifier. A second input of the operational amplifier is coupled to an output of a second, whose output represents the voltage across the measurement cell. The first operational amplifier is connected in a voltage follower configuration, in which the amplifier will adjust its output until the voltage appearing at its second input is equal to the commanded voltage appearing at its first input.

US 2015/362455 A1 describes a concentration measuring device or test meter with an analyte test sensor strip mounted thereto that is used to measure the presence or concentration of an analyte in a biological fluid. To ensure that the proper voltage potential is applied to the counter electrode, the test meter includes circuitry that ensures that a voltage potential (or absolute potential difference) applied to the counter sense trace is the same as the desired voltage potential (or absolute potential difference) at the counter electrode.

US 2015/330935 A1 discusses a microprocessor that is able to adjust an applied potential to maintain the desired or expected voltage at a measurement electrode.

### BRIEF DESCRIPTION OF THE DRAWINGS

The accompanying drawings, which are incorporated herein and constitute part of this specification, illustrate presently preferred embodiments of the invention, and, together with the general description given above and the detailed description given below, serve to explain features of the invention, in which:
FIG. 1 is a simplified exploded perspective view of an electrochemical-based analytical test strip;
FIG. 2 is a simplified top view of a patterned conductor layer and substrate layer of the electrochemical-based analytical test strip of FIG. 1 with the location of an enzymatic reagent layer depicted by dashed lines;
FIG. 3 is a simplified top view of a hand-held test meter according to an embodiment of the present invention;
FIG. 4 is a simplified block diagram of the hand-held test meter of FIG. 4; and
FIG. 5 is a simplified schematic diagram of an automatic bias drive adjustment circuit block for a single working electrode (WE) and reference electrode (REF) pair as can be employed in embodiments of the present invention.

### DETAILED DESCRIPTION OF ILLUSTRATIVE EMBODIMENTS

The following detailed description should be read with reference to the drawings, in which like elements in different drawings are identically numbered. The drawings, which are not necessarily to scale, depict exemplary embodiments for the purpose of explanation only and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable tolerance that allows a component or collection of components to function for its intended purpose as described herein.

An electrochemical-based analytical test strip for use with a hand-held test meter in the determination of an analyte (such as glucose) in a bodily fluid sample (e.g., a whole blood sample) includes an electrically insulating base layer, a patterned electrically conductive layer disposed on the electrically insulating base layer, an enzymatic reagent layer, a patterned spacer layer; and a top layer. The patterned electrically conductive layer includes at least one electrode (for example, two electrodes, namely a working electrode and a reference electrode), at least one electrode voltage sensing connection configured to sense voltage at the least one electrode (e.g., a working electrode voltage sensing connection and a reference electrode voltage sensing connection), at least one electrode track, and at least one electrode voltage sensing connection track. Moreover, the electrode voltage sensing connections are configured for operable communication of a sensed electrode voltage to an associated hand-held test meter via the at least one electrode voltage sensing connection.

Electrochemical-based analytical test strips described herein are beneficial in that the electrode voltage sensing connections and electrode voltage sensing connection tracks can be employed by an associated hand-held test meter (described herein) to measure deleterious voltage drop(s) on the electrochemical-based analytical test strip. Such measured voltage drop(s) can then be employed by the hand-held test meter to automatically (for example, dynamically) adjust a voltage bias drive(s) applied to the electrochemical-based analytical test strip by the hand-held test meter. Such automatic adjustments result in the electrode track resistance and dimensional tolerance being beneficially less critical to accurate use of embodiments of electrochemical-based analytical test strips and hand-held test meters of the present invention.

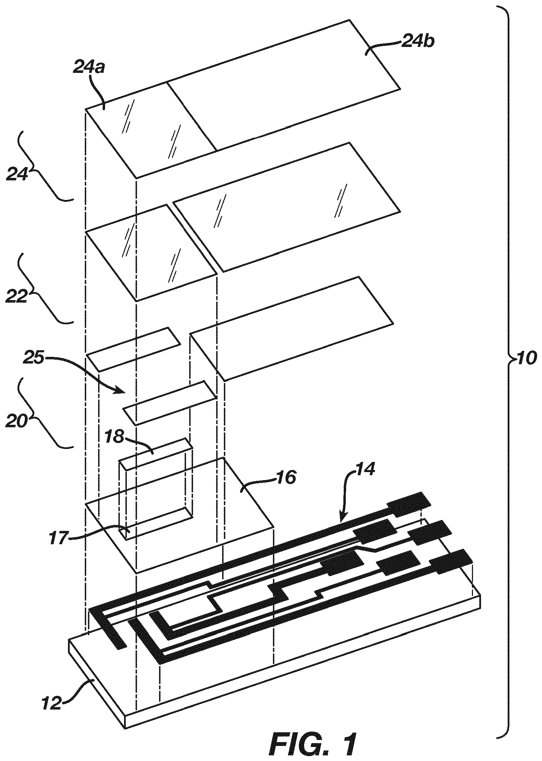

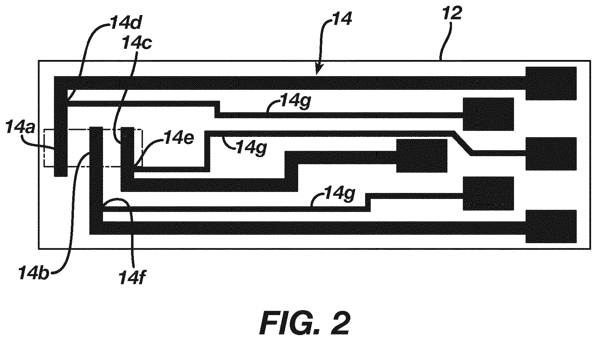

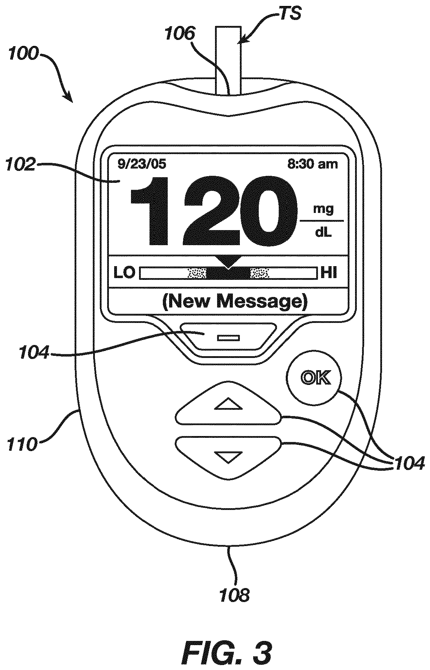

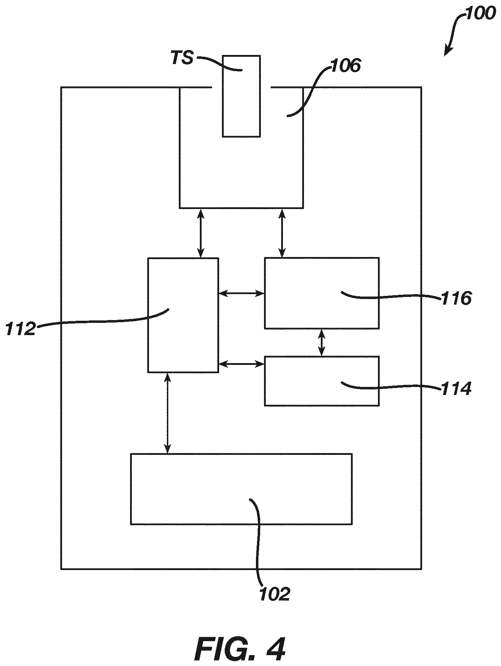

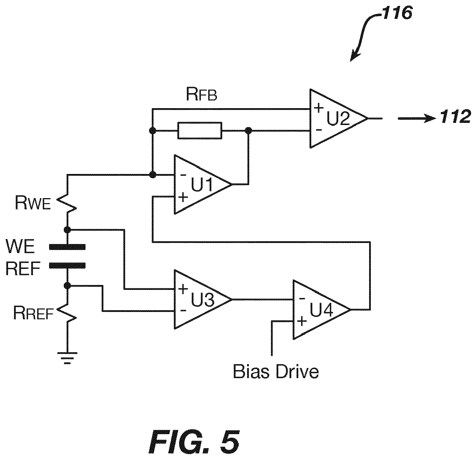

FIG. 1 is a simplified exploded perspective view of an electrochemical-based analytical test strip 10. FIG. 2 is a simplified top view of a patterned conductor layer and substrate layer of electrochemical-based analytical test strip 10 with the location of an enzymatic reagent layer depicted by dashed lines. FIG. 3 is a simplified top view of a hand-held test meter 100 according to an embodiment of the present invention. FIG. 4 is a simplified block diagram of hand-held test meter 100. FIG. 5 is a simplified schematic diagram of an electrode bias adjustment circuit block for a single working electrode (WE) and reference electrode (REF) pair as can be employed in hand-held test meter embodiments of the present invention.

Referring to FIGs. 1 and 2, electrochemical-based analytical test strip 10 for the determination of an analyte (such as glucose) in a bodily fluid sample (for example, a whole blood sample) includes an electrically-insulating base layer 12, a patterned electrically conductive layer 14, a patterned insulation layer 16 with opening 17 therethrough, an enzymatic reagent layer 18, a patterned spacer layer 20, and a top layer consisting of a hydrophilic sub-layer 22 and a top tape 24 with portions 24a and 24b.

In the embodiment of FIGs. 1 and 2, at least the patterned spacer layer and top layer define a sample-receiving chamber 25 within electrochemical-based analytical test strip 10.

An electrochemical-based analytical test strip 10 is configured for use with a hand-held test meter (e.g., a hand-held test meter described herein) in the determination of an analyte in a bodily fluid sample, the electrochemical-based analytical test strip. See, for example, the hand-held test meter described with respect to FIGs 3, 4 and 5.

Electrically-insulating base layer 12 can be any suitable electrically-insulating base layer known to one skilled in the art including, for example, a nylon base layer, a polycarbonate base layer, a polyimide base layer, a polyvinyl chloride base layer, a polyethylene base layer, a polypropylene base layer, a glycolated polyester (PETG) base layer, or a polyester base layer. The electrically-insulating base layer can have any suitable dimensions including, for example, a width dimension of about 5 mm, a length dimension of about 27 mm and a thickness dimension of about 0.5 mm.

Electrically-insulating base layer 12 provides structure to electrochemical-based analytical test strip 10 for ease of handling and also serves as a base for the application (e.g., printing or deposition) of subsequent layers (e.g., a patterned electrically conductor layer and an enzymatic reagent formed by ink jet printing or screen printing of an enzymatic reagent layer described herein).

Patterned electrically conductive layer 14 is disposed on the electrically-insulating base layer 12 and includes a first electrode 14a, a second electrode 14b and a third electrode 14c. First electrode 14a, second electrode 14b and third electrode 14c can be, for example, configured as a counter/reference electrode, a first working electrode and a second working electrode, respectively. Therefore, the second and third electrodes are also referred to herein as working electrodes 14b and 14c and the first electrode as counter electrode 14a. Although, for the purpose of explanation only, electrochemical-based analytical test strip 10 is depicted as including a total of three electrodes, embodiments of electrochemical-based analytical test strips can include any suitable number of electrodes.

Patterned electrically conductive layer 14 also includes a first electrode voltage sensing connection 14d, a second electrode voltage sensing connection 14e and a third electrode voltage sensing connection 14f configured to sense electrode voltage at the counter/reference electrode, first working electrode and second working electrode respectively.

Patterned electrically conductive layer 14 also includes a plurality of electrode connection tracks 14g configured for operable communication of a sensed electrode voltage to a hand-held test meter.

Patterned electrically conductive layer 14 can be formed of any suitable conductive material including, for example, electrically conducting carbon-based materials including carbon inks. It should be noted that patterned electrically conductive layers employed in electrochemical-based analytical test strips described herein can take any suitable shape and be formed of any suitable materials including, for example, metal materials and conductive carbon materials.

Referring to FIGs. 1 and 2, the disposition of first electrode 14a, second electrode 14b and third electrode 14c and enzymatic reagent layer 18 are such that electrochemical-based analytical test strip 10 is configured for the electrochemical determination of an analyte (such as glucose) in a bodily fluid sample (such as a whole blood sample) that has filled sample-receiving chamber 25.

Enzymatic reagent layer 18 is disposed on at least a portion of patterned electrically conductor layer 14 (see FIG. 2 wherein the disposition of enzymatic reagent layer 18 is depicted by dashed lines). Once apprised of the present disclosure, one skilled in the art will recognize that a variety of suitable enzymatic reagents are known to one skilled in the art. Further details regarding reagent layers in general, and electrochemical-based analytical test strips in general, are in U.S. Patent Nos. 6,241,862 and 6,733,655.

Referring to FIGs. 1 and 2, patterned insulation layer 16 can be formed of any suitable electrically-insulating dielectric material including commercially available screen-printable dielectric inks.

Patterned spacer layer 20 can be formed, for example, from a screen-printable pressure sensitive adhesive commercially available from Apollo Adhesives, Tamworth, Staffordshire, UK. In the embodiment of FIG. and 2, patterned spacer layer 20 defines outer walls of the sample-receiving chamber 25. Patterned spacer layer 20 can have a thickness of, for example, approximately 110 microns, be electrically nonconductive, and be formed of a polyester material with top and bottom side acrylic-based pressure sensitive adhesive.

Top layer 24 can be, for example, a clear film with hydrophilic properties that promote wetting and filling of electrochemical-based analytical test strip 10 by a fluid sample (e.g., a whole blood sample). Such clear films are commercially available from, for example, 3M of Minneapolis, Minnesota U.S.A. and Coveme (San Lazzaro di Savena, Italy). Top layer 24 can be, for example, a polyester film coated with a surfactant that provides a hydrophilic contact angle less than 10 degrees. Top layer 24 can also be a polypropylene film coated with a surfactant or other surface treatment. In such a circumstance, the surfactant coating serves as hydrophilic sub-layer 22. Top layer 24 can have a thickness, for example, of approximately 100µm.

Electrochemical-based analytical test strip 10 can be manufactured, for example, by the sequential aligned formation of the layers depicted in FIG 1. Any suitable techniques known to one skilled in the art can be used to accomplish such sequential aligned formation, including, for example, screen printing, ink-jet printing, photolithography, photogravure, chemical vapour deposition and tape lamination techniques. However, enzymatic reagents are particularly beneficial in that they can be formulated as aqueous compositions suitable for relatively low-cost and otherwise conventional ink jet and screen printing techniques.

Electrochemically-based analytical test strip 10 is configured such that the various electrode voltages can be sensed (using the electrode's associated electrode voltage sensing connections 14d, 14e and 14f, associated electrode connection tracks 14g and an associated hand-held test meter). Using the voltages sensed at the electrodes themselves, any deleterious voltage drops can be measured and a bias drive from an associated hand-held test meter increased to compensate for the deleterious voltage drop. This can be done, for example, dynamically using a closed loop error amplifier circuit (or as elsewhere described herein).

A hand-held test meter for use with an electro-chemical-based analytical test strip in the determination of an analyte in a bodily fluid sample according to embodiments of the present invention includes a housing, a strip port connector disposed at least partially within the housing and configured to receive an electro-chemical based analytical test strip, a micro-controller disposed in the housing and configured to generate a micro-controller command signal, an electrode bias drive circuit block disposed in the housing and configured to generate a bias drive signal based on the micro-controller command signal, and an automatic bias drive adjustment circuit block disposed in the housing and configured to receive at least one sensed electrode voltage and to adjust a bias drive signal from the electrode bias drive circuit block based on the least one sensed electrode voltage to create an adjusted bias drive signal. The hand-held test meter according to the present invention is defined in claim 1.

Referring to FIGs. 3, 4 and 5, hand-held test meter 100 includes a display 102, a plurality of user interface buttons 104, a strip port connector 106, a USB interface 108, and a housing 110 (see FIG. 3). Referring to FIG. 4 in particular, hand-held test meter 100 also includes a micro-controller block 112, an electrode bias drive circuit block 114 disposed in the housing and configured to generate a bias drive signal based on the micro-controller command signal, and an automatic bias drive adjustment circuit block 116 disposed in the housing and configured to receive at least one sensed electrode voltage and to adjust a bias drive signal from the electrode bias drive circuit block based on the least one sensed electrode voltage to create an adjusted bias drive signal, and other electronic components (not shown in the FIGs.) for applying an electrical bias (e.g., an alternating current [AC] and/or direct current [DC] bias) to an electrochemical-based analytical test strip (labeled TS in FIGs. 3 and 4), and also for measuring an electrochemical response (e.g., plurality of test current values, phase, and/or magnitude) and determining an analyte or characteristic based on the electrochemical response. To simplify the current descriptions, the figures do not depict all such electronic circuitry.

Display 102 can be, for example, a liquid crystal display or a bi-stable display configured to show a screen image. An example of a screen image during the determination of an analyte in a bodily fluid sample may include a glucose concentration, a date and time, an error message, and a user interface for instructing a user how to perform a test. Examples of screen images during use of the operating range test strip simulation circuit block may be an image reporting that a hand-held test meter operating range test passed, or an image reporting that the hand-held test meter operating range test has resulted in an error.

Strip port connector 106 is configured to operatively interface with an electrochemical-based analytical test strip TS, such as an electrochemical-based analytical test strip configured for the determination of hematocrit and/or glucose in a whole blood sample. Therefore, the electrochemical-based analytical test strip is configured for operative insertion into strip port connector 106 and to operatively interface with micro-controller block 112 via, for example, suitable electrical contacts, wires, electrical interconnects or other structures known to one skilled in the art.

USB Interface 108 can be any suitable interface known to one skilled in the art. USB Interface 108 is an electrical component that is configured to power and provide a data line to hand-held test meter 100.

Micro-controller block 112 also includes a memory sub-block that stores suitable algorithms for the determination of an analyte based on the electrochemical response of an analytical test strip and to also determine a characteristic (e.g., hematocrit) of the introduced bodily fluid sample. Micro-controller block 112 is disposed within housing 110 and can include any suitable micro-controller and/or micro-processer known to those skilled in the art. Suitable micro-controllers include, but are not limited to, micro-controllers available commercially from Texas Instruments (Dallas, Texas, USA) under the MSP430 series of part numbers; from ST MicroElectronics (Geneva, Switzerland) under the STM32F and STM32L series of part numbers; and Atmel Corporation (San Jose, California, USA) under the SAM4L series of part numbers).

Referring, in particular, to FIG 5, an exemplary, but non-limiting, automatic bias drive adjustment circuit block 116 for a single working electrode and associated counter/reference electrode is depicted using conventional electronic component symbols. In FIG. 5, WE is a working electrode, REF is a counter/reference electrode, R represents a resistor, and U represents an amplifier. Automatic bias drive adjustment circuit block 116 can be generally considered a closed loop error amplifier circuit.

The operation of hand-held test meter 100 is now described with reference to FIGs. 3, 4 and, in particular, 5. During operation, system micro-controller 112 commands a required bias onto the bias drive signal (see FIG. 5), via an electrode bias drive circuit block 114 (for example, via a DAC serving as an electrode bias drive circuit block). R_{we} and R_{ref} represent the additional resistance in the circuit introduced by the electrode tracks. U3 is a difference amplifier with a gain of x1 that provides a measurement of the actual voltage across an electrode WE. U4 is an error amplifier that compares the measured electrode voltage against the commanded bias drive value. This error amplifier U4 typically has a high gain (at least 100).

U1 is, for example, a trans-impedance amplifier that uses U4 output to both drive a bias across the electrodes (WE and REF in FIG 5), and to measure a working electrode WE current. The U4 drive is effectively slightly above the required bias drive, compensating for the voltage drop across the electrode track resistance. All the current flowing through the working electrode WE is also pushed through R_{FB}, and therefore an accurate measurement of the voltage across R_{FB} provides an indication of working electrode current. Depending upon the maximum working electrode current, R_{FB} is scaled to provide maximum ADC input for maximum WE current. The value R_{FB} be, for example, approximately around 100K ohm for a patterned electrically conductive layer formed of carbon. U2 is a difference amplifier with a gain of x1, and provides an accurate measurement of the voltage across R_{FB} to the output of U2. U2 output then feeds into an ADC input of the system microcontroller.

Once apprised of the present disclosure, one skilled in the art will recognize that automatic bias drive circuit blocks employed in hand-held test meters according to embodiments of the present invention can take various forms and are not limited to the embodiment depicted in FIG. 5, but only by the appended claims.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that devices and compositions of matter within the scope of these claims be covered thereby

## Claims

1. A hand-held test meter (100) for use with an electro-chemical-based analytical test strip (10) in the determination of an analyte in a bodily fluid sample, the hand-held test meter comprising:
a housing (110);
a strip port connector (106) disposed at least partially within the housing and configured to receive an electro-chemical based analytical test strip;
a micro-controller (112) disposed in the housing and configured to generate a micro-controller command signal, the micro-controller comprising an analogue-to-digital converter input;
an electrode bias drive circuit block (114) disposed in the housing and configured to generate a bias drive signal based on the micro-controller command signal;
an automatic bias drive adjustment circuit block (116) disposed in the housing and configured to receive at least one sensed electrode voltage and to adjust a bias drive signal from the electrode bias drive circuit block based on the at least one sensed electrode voltage to create an adjusted bias drive signal, wherein the automatic bias drive adjustment circuit block comprises:
a first difference amplifier (U3) configured to provide the at least one sensed electrode voltage;
an error amplifier (U4) configured to compare the at least one sensed electrode voltage to the generated bias drive signal;
a trans-impedance amplifier (U1) configured to use the output of the error amplifier (U4) to both drive the adjusted bias drive signal and to measure at least one sensed electrode current;
a resistor (R_{FB}) across the trans-impedance amplifier (U1);
a second difference amplifier (U2) configured to provide a measurement of the voltage across the resistor (R_{FB}) to an output of the second difference amplifier (U2),
wherein the second difference amplifier (U2) is further configured to feed into the analogue-to-digital converter input of the micro-controller.

2. The hand-held test meter of claim 1 wherein the electrode bias drive circuit block (114) is a Digital-to-Analog Converter (DAC).

3. The hand-held test meter of claim 1 wherein the hand-held test meter (100) is configured for the determination of glucose in a whole blood sample applied to the electrochemical-based analytical test strip (10).

4. A system comprising the hand-held test meter (100) of claim 1 and the electrochemical-based analytical test strip (10), for use in the determination of an analyte in a bodily fluid sample, the electrochemical-based analytical test strip comprising:
an electrically insulating base layer (12);
a patterned electrically conductive layer (14) disposed on the electrically insulating base layer and including at least one electrode;
an enzymatic reagent layer (18) disposed on the at least one electrode;
a patterned spacer layer (20); and
a top layer (22; 24); wherein the patterned electrically conductive layer includes:
a plurality of electrodes (14a; 14b; 14c); and
at least one electrode voltage sensing connection (14d; 14e; 14f) configured to sense electrode voltage of at least one of the plurality of electrodes; and
a plurality of electrode tracks; and
at least one electrode voltage sensing connection track configured for operable communication of a sensed electrode voltage to a hand-held test meter.

5. The system of claim 4 wherein the plurality of electrodes includes:
a first working electrode;
a second working electrode; and
a reference electrode.

6. The system of claim 5 wherein the at least one electrode voltage sensing connections includes:
a first electrode voltage sensing connection in electrical communication with the first working electrode;
a second electrode voltage sensing connection in electrical connection with the second working electrode; and
a third electrode voltage sensing connection in electrical connection with the reference electrode.

7. The system of claim 4 wherein the patterned electrically conductive layer comprises a carbon-containing material.

8. The system of claim 4 wherein each of the plurality of voltage sensing connections are electrically connected to a single one of the electrodes at a distance of less than 10 mm.

9. The system of claim 4 wherein each of the plurality of electrode voltage sensing connections includes:
an electrode voltage sensing track; and
an electrode voltage sensing pad.

10. The system of claim 4 wherein the bodily fluid sample is a whole blood sample and the analyte is glucose.

## Patentansprüche

1. Tragbares Messgerät (100) zur Verwendung mit einem elektrochemisch basierten Analyseteststreifen (10) bei der Bestimmung eines Analyten in einer Körperflüssigkeitsprobe, wobei das tragbare Messgerät Folgendes umfasst:
ein Gehäuse (110);
einen Streifenanschlussverbinder (106), der zumindest teilweise innerhalb des Gehäuses angeordnet ist und zur Aufnahme eines elektrochemisch basierten Analyseteststreifens ausgelegt ist;
einen Mikrocontroller (112), der in dem Gehäuse angeordnet ist und zum Erzeugen eines Mikrocontroller-Befehlssignals ausgelegt ist, wobei der Mikrocontroller einen Analog-Digital-Wandler-Eingang umfasst;
einen Elektrodenvorspannungsansteuerung-Schaltungsblock (114), der in dem Gehäuse angeordnet ist und zum Erzeugen eines Vorspannungsansteuersignals basierend auf dem Mikrocontroller-Befehlssignal ausgelegt ist;
einen Automatische-Vorspannungsansteuerungsanpassung-Schaltungsblock (116), der in dem Gehäuse angeordnet ist und ausgelegt ist zum Empfangen mindestens einer erfassten Elektronenspannung und zum Anpassen eines Vorspannungsansteuersignals von dem Elektrodenvorspannungsansteuerung-Schaltungsblock basierend auf der mindestens einen erfassten Elektrodenspannung, um ein angepasstes Vorspannung Ansteuersignal zu erzeugen, wobei der Automatische-Vorspannungsansteuerungsanpassung-Schaltungsblock Folgendes umfasst:
einen ersten Differenzverstärker (U3), ausgelegt zum Bereitstellen der mindestens einen erfassten Elektrodenspannung;
einen Fehlerverstärker (U4), ausgelegt zum Vergleichen der mindestens einen erfassten Elektrodenspannung mit dem erzeugten Vorspannungsansteuersignal;
einen Transimpedanzverstärker (U1), ausgelegt zum Verwenden der Ausgabe des Fehlerverstärkers (U4) sowohl zum Ansteuern des angepassten Vorspannungsansteuersignals als auch zum Messen mindestens eines erfassten Elektrodenstroms;
einen Widerstand (R_{FB}) über dem Transimpedanzverstärker (U1) ;
einen zweiten Differenzverstärker (U2), ausgelegt zum Liefern einer Messung der Spannung an dem Widerstand (R_{FB}) an einen Ausgang des zweiten Differenzverstärkers (U2),
wobei der zweite Differenzverstärker (U2) ferner zum Einspeisen in den Analog-Digital-Wandler-Eingang des Mikrocontrollers ausgelegt ist.

2. Tragbares Messgerät nach Anspruch 1, wobei der Elektrodenvorspannungsansteuerung-Schaltungsblock (114) ein Digital-Analog-Wandler (DAC) ist.

3. Tragbares Messgerät nach Anspruch 1, wobei das tragbare Messgerät (100) zur Bestimmung von Glucose in einer auf den elektrochemisch basierten Analyseteststreifen (10) aufgebrachten Vollblutprobe ausgelegt ist.

4. System, umfassend das tragbare Messgerät (100) nach Anspruch 1 und den elektrochemisch basierten Analyseteststreifen (10) zur Verwendung bei der Bestimmung eines Analyten in einer Körperflüssigkeitsprobe, wobei der elektrochemisch basierte Analyseteststreifen Folgendes umfasst:
eine elektrisch isolierende Basisschicht (12);
eine strukturierte elektrisch leitfähige Schicht (14), die auf der elektrisch isolierenden Basisschicht angeordnet ist und mindestens eine Elektrode aufweist;
eine Enzymreagensschicht (18), die auf der mindestens eine Elektrode angeordnet ist;
eine strukturierte Abstandshalterschicht (20); und
eine Deckschicht (22; 24);
wobei die strukturierte elektrisch leitfähige Schicht Folgendes aufweist:
mehrere Elektroden (14a; 14b; 14c); und
mindestens eine Elektrodenspannungserfassungsverbindung (14d; 14e; 14f), ausgelegt zum Erfassen einer Elektrodenspannung von mindestens einer der mehreren Elektroden; und
mehrere Elektrodenspuren; und
mindestens eine Elektrodenspannungserfassungsverbindungsspur, ausgelegt zur betriebsfähigen Kommunikation einer erfassten Elektrodenspannung zu einem tragbaren Messgerät.

5. System nach Anspruch 4, wobei die mehreren Elektroden Folgendes aufweisen:
eine erste Arbeitselektrode;
eine zweite Arbeitselektrode; und
eine Referenzelektrode.

6. System nach Anspruch 5, wobei die mindestens eine Elektrodenspannungserfassungsverbindung Folgendes aufweist:
eine erste Elektrodenspannungserfassungsverbindung in elektrischer Kommunikation mit der ersten Arbeitselektrode;
eine zweite Elektrodenspannungserfassungsverbindung in elektrischer Verbindung mit der zweiten Arbeitselektrode;
eine dritte Elektrodenspannungserfassungsverbindung in elektrischer Verbindung mit der Referenzelektrode.

7. System nach Anspruch 4, wobei die strukturierte elektrisch leitfähige Schicht ein kohlenstoffhaltiges Material umfasst.

8. System nach Anspruch 4, wobei jede der Spannungserfassungsverbindungen elektrisch mit einer einzelnen der Elektroden in einem Abstand von weniger als 10 mm verbunden ist.

9. System nach Anspruch 4, wobei jede der mehreren Elektrodenspannungserfassungsverbindungen Folgendes aufweist:
eine Elektrodenspannungserfassungsspur; und
ein Elektrodenspannungserfassungspad.

10. System nach Anspruch 4, wobei es sich bei der Körperflüssigkeitsprobe um eine Vollblutprobe handelt und es sich bei dem Analyten um Glucose handelt.

## Revendications

1. Appareil de mesure portatif (100) à utiliser avec une bandelette réactive analytique de type électrochimique (10) pour la détermination d'un analyte dans un échantillon de fluide corporel, l'appareil de mesure portatif comprenant :
un boîtier (110) ;
un connecteur de port de bandelette (106) disposé au moins partiellement à l'intérieur du boîtier et configuré pour recevoir une bandelette réactive analytique de type électrochimique ;
un microcontrôleur (112) disposé dans le boîtier et configuré pour générer un signal de commande de microcontrôleur ; le microcontrôleur comprenant une entrée de convertisseur analogique-numérique ;
un bloc de circuit de commande de polarisation d'électrode (114) disposé dans le boîtier et configuré pour générer un signal de commande de polarisation sur la base du signal de commande de microcontrôleur ;
un bloc de circuit d'ajustement de commande de polarisation automatique (116) disposé dans le boîtier et configuré pour recevoir au moins une tension d'électrode détectée et pour ajuster un signal de commande de polarisation provenant du bloc de circuit de commande de polarisation d'électrode sur la base d'au moins une tension d'électrode détectée afin de créer un signal de commande de polarisation ajusté, le bloc de circuit d'ajustement de commande de polarisation automatique comprenant :
un premier amplificateur de différence (U3) configuré pour fournir l'au moins une tension d'électrode détectée ;
un amplificateur d'erreur (U4) configuré pour comparer l'au moins une tension d'électrode détectée au signal de commande de polarisation généré ;
un amplificateur de trans-impédance (U1) configuré pour utiliser la sortie de l'amplificateur d'erreur (U4) à la fois pour commander le signal de commande de polarisation ajusté et pour mesurer au moins un courant d'électrode détecté ;
une résistance (R_{FB}) aux bornes de l'amplificateur de trans-impédance (U1) ;
un second amplificateur de différence (U2) configuré pour fournir une mesure de la tension aux bornes de la résistance (R_{FB}) à une sortie du second amplificateur de différence (U2),
le second amplificateur de différence (U2) étant en outre configuré pour alimenter l'entrée du convertisseur analogique-numérique du microcontrôleur.

2. Appareil de mesure portatif selon la revendication 1, le bloc de circuit de commande de polarisation d'électrode (114) étant un convertisseur numérique-analogique (CNA).

3. Appareil de mesure portatif selon la revendication 1, l'appareil de mesure portatif (100) étant configuré pour la détermination du glucose dans un échantillon de sang total appliqué à la bandelette réactive analytique de type électrochimique (10).

4. Système comprenant l'appareil de mesure portatif (100) selon la revendication 1 et la bandelette réactive analytique de type électrochimique (10), pour la détermination d'un analyte dans un échantillon de fluide corporel, la bandelette réactive analytique de type électrochimique comprenant :
une couche de base électriquement isolante (12) ;
une couche conductrice d'électricité à motifs (14) disposée sur la couche de base électriquement isolante et comprenant au moins une électrode ;
une couche de réactif enzymatique (18) disposée sur l'au moins une électrode ;
une couche d'espacement à motifs (20) ; et
une couche supérieure (22 ; 24) ;
la couche conductrice d'électricité à motifs comprenant :
une pluralité d'électrodes (14a ; 14b ; 14c) ; et
au moins une connexion de détection de tension d'électrode (14d ; 14e ; 14f) configurée pour détecter la tension d'électrode d'au moins une de la pluralité d'électrodes ; et
une pluralité de pistes d'électrodes ; et
au moins une piste de connexion de détection de tension d'électrode configurée pour la communication opérationnelle d'une tension d'électrode détectée à un appareil de mesure portatif.

5. Système selon la revendication 4, la pluralité d'électrodes comprenant :
une première électrode de travail
une deuxième électrode de travail ; et
une électrode de référence.

6. Système selon la revendication 5, l'au moins une connexion de détection de tension d'électrode comprenant :
une première connexion de détection de tension d'électrode en communication électrique avec la première électrode de travail ;
une deuxième connexion de détection de tension d'électrode en communication électrique avec la deuxième électrode de travail ; et
une troisième connexion de détection de tension d'électrode en connexion électrique avec l'électrode de référence.

7. Système selon la revendication 4, la couche conductrice d'électricité à motifs comprenant un matériau contenant du carbone.

8. Système selon la revendication 4, chacune de la pluralité de connexions de détection de tension étant connectée électriquement à une seule des électrodes à une distance inférieure à 10 mm.

9. Système selon la revendication 4, chacune de la pluralité de connexions de détection de tension d'électrode comprenant :
une piste de détection de tension d'électrode ; et
une plage de détection de tension d'électrode.

10. Système selon la revendication 4, l'échantillon de fluide corporel étant un échantillon de sang total et l'analyte étant le glucose.
